(19) Europäisches Patentamt
European Patent Office
Office européen des brevets

(11) **EP 2 229 118 B1**

(12) **EUROPÄISCHE PATENTSCHRIFT**

(45) Veröffentlichungstag und Bekanntmachung des Hinweises auf die Patenterteilung:
**18.12.2013 Patentblatt 2013/51**

(21) Anmeldenummer: **08870931.6**

(22) Anmeldetag: **29.12.2008**

(51) Int Cl.:
***A61B 19/00*** *(2006.01)*

(86) Internationale Anmeldenummer:
**PCT/EP2008/068312**

(87) Internationale Veröffentlichungsnummer:
**WO 2009/089992 (23.07.2009 Gazette 2009/30)**

(54) **SPULENANORDNUNG ZUR FÜHRUNG EINES MAGNETISCHEN ELEMENTS IN EINEM ARBEITSRAUM**

COIL ARRANGEMENT FOR GUIDING A MAGNETIC ELEMENT IN A WORKING SPACE

DISPOSITIF DE BOBINES DESTINÉ AU GUIDAGE D'UN ÉLÉMENT MAGNÉTIQUE DANS UN ESPACE DE TRAVAIL

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **17.01.2008 DE 102008004871**

(43) Veröffentlichungstag der Anmeldung:
**22.09.2010 Patentblatt 2010/38**

(73) Patentinhaber: **Siemens Aktiengesellschaft**
**80333 München (DE)**

(72) Erfinder: **REINSCHKE, Johannes**
**90419 Nürnberg (DE)**

(56) Entgegenhaltungen:
**WO-A-99/11189        WO-A-2006/092421**
**DE-B3- 10 340 925**

**Beschreibung**

Die Erfindung betrifft eine Spulenanordnung zur berührungslosen Führung eines magnetischen Elements, insbesondere einer Endoskopiekapsel, in einem Arbeitsraum.

Die Verwendung von Endoskopen und Kathetern findet in der Medizin zur Diagnose oder zur Behandlung des Inneren eines Patienten immer breitere Anwendung. Die Instrumente werden über Körperöffnungen oder Schnitte in den Körper eingeführt und lassen sich von außen geführt in einer Längsrichtung verschieben, wofür eine mechanische Verbindung zum Instrument notwendig ist. Bei der Vorwärtsbewegung des Instruments in den Körper hinein treten jedoch an Kurven oder Verzweigungen in der Regel Schwierigkeiten bei der Navigation auf in der Form, dass der Bediener das Instrument ggf. durch mehrfaches Probieren in die gewollte Richtung dirigieren muss und eine Stützkraft vom Gewebe auf das Instrument für die weitere Navigation erforderlich ist. Dies ist für den Bediener mit höherem zeitlichem Aufwand und für den Patienten mit Schmerzen verbunden. Im schlechtesten Fall ist nicht auszuschließen, dass die Führung in die geplante Richtung gar nicht gelingt oder das Risiko der Gewebeperforation eintritt. Weiterhin kann es im Fall der Endoskopie von Interesse sein, den mit einer Kamera ausgestatteten Endoskopiekopf in bestimmte Richtungen zu drehen, um z.B. die Schleimhaut in einem Abschnitt des Magen-Darmtrakts vollständig zu sehen. Dies ist mit heutigen Katheterendoskopen nur bedingt möglich, weil die Katheterspitze nur eingeschränkt beweglich ist. Darüber hinaus weisen übliche Katheterendoskope den Nachteil auf, dass entfernt liegende innere Organe nur schwer oder gar nicht erreichbar sind.

Die passive, durch die natürliche Peristaltik des Magen-Darmtraktes bewegte Endoskopiekapsel hat die genannten Nachteile der Katheterendoskope nicht, ist allerdings auch nicht navigierbar, d.h. die gezielte Sichtung bestimmter Stellen im Inneren das Magen-Darm-Traktes ist nicht möglich. Es wurden daher magnetische Navigations- bzw. Führungssysteme vorgeschlagen, die eine katheter- bzw. drahtlose Führung von Endoskopiekapseln, die ein magnetisches Dipolmoment enthalten, ermöglichen. Eine katheter- oder drahtlose Führung wird nachstehend auch als "berührungslos" bezeichnet.

Die DE 103 40 925 B3 und die WO 2006/092421 A1 beschreiben jeweils eine Magnetspulenanordnung bestehend aus 14 Einzelspulen zur Navigation einer Endoskopiekapsel, einer Videokapsel oder einer sonstigen Sonde. Die Kapsel ist hierbei mit einem magnetischen Element, bspw. einem Permanent- oder Ferromagneten, ausgestattet. Die Magnetspulenanordnung erzeugt Magnetfeldkomponenten $B_x$, $B_y$, $B_z$ entlang der Achsen x, y, z eines kartesischen Koordinatensystems sowie magnetische Gradientenfelder, die eine berührungslose Führung der Endoskopiekapsel ermöglichen.

Hierbei wird ausgenutzt, dass sich das magnetische Element, d.h. ein Körper mit einem magnetischen Dipolmoment $\vec{m}$, parallel zur Richtung des magnetischen Feldes $\vec{B}$ bestehend aus den Magnetfeldkomponenten $B_x$, $B_y$, $B_z$ in Richtung der Achsen des kartesischen Koordinatensystems auszurichten versucht. Da das magnetische Element fest mit der Endoskopiekapsel verbunden ist, kann so die Orientierung der Kapsel beeinflusst werden. Zusätzlich wirkt ausgelöst durch die magnetischen Gradientenfelder $\partial B_x/\partial x$ etc. eine Kraft $\vec{F} = \underline{\underline{G}} \cdot \vec{m}$ mit einer die Gradientenfelder umfassenden Gradientenmatrix $\underline{\underline{G}}$ auf das magnetische Dipolmoment $\vec{m}$ gemäß

$$\vec{F} \;=\; \underline{\underline{G}} \cdot \vec{m} \;=\; \begin{pmatrix} \partial B_x / \partial x & \partial B_x / \partial y & \partial B_x / \partial z \\ \partial B_y / \partial x & \partial B_y / \partial y & \partial B_y / \partial z \\ \partial B_z / \partial x & \partial B_z / \partial y & \partial B_z / \partial z \end{pmatrix} \cdot \vec{m}\,.$$

Die Gradientenmatrix G ist aufgrund der Maxwell-Gleichungen rot $\vec{B}$ = 0 und div $\vec{B}$ = 0 symmetrisch und spurfrei, d.h. sie enthält mit $\partial B_x/\partial y$ (= $\partial B_y / \partial x$), $\partial B_x / \partial z$ (= $\partial B_z / \partial x$), $\partial B_y / \partial z$ (= $\partial B_z / \partial y$) und zweien der drei Diagonalelemente (bspw. $\partial B_x / \partial x$ und $\partial B_y / \partial y$) fünf unabhängige Gradientenfelder.

Durch eine gezielte Ansteuerung der Einzelspulen der Magnetspulenanordnung können das Magnetfeld $\vec{B}$ und die Gradientenfelder beliebig eingestellt werden. Es ist damit zum Einen möglich, das magnetische Element zu drehen und es somit beliebig in einem Arbeitsraum A innerhalb der Magnetspulenanordnung auszurichten. Zum Anderen ist es möglich, eine Kraft $\vec{F}$ auf das magnetische Element auszuüben, um es zusätzlich zur Drehung translatorisch zu verschieben. Hierzu werden acht quasi-statische magnetische Freiheitsgrade realisiert, nämlich die Magnetfeldkomponenten $B_x$, $B_y$, $B_z$ sowie zwei der drei Einträge der Diagonalelemente (bspw. $\partial B_x / \partial x$ und $\partial B_y / \partial y$ ) und drei der Nebendiagonalelemente (bspw. $\partial B_x / \partial y$, $\partial B_z / \partial x$, $\partial B_z / \partial y$) der Gradientenmatrix $\underline{\underline{G}}$.

Die in DE 103 40 925 B3 und WO 2006/092421 A1 beschriebenen Systeme haben den Nachteil, dass sie aufgrund der dort benötigten 14 einzeln angesteuerten Spulen wegen der hohen Zahl an Spulen und Leistungsverstärkern relativ kostenintensiv in Herstellung und Installation sind.

Aufgabe der Erfindung ist es daher, ein kostengünstigeres magnetisches Führungssystem bestehend aus einer Spulenanordnung und mehreren, den Spulen zugeordneten Leistungsverstärkern anzugeben.

Diese Aufgabe wird durch die in dem unabhängigen Anspruch angegebene Erfindung gelöst. Vorteilhafte Ausgestaltungen ergeben sich aus den abhängigen Ansprüchen.

Die vorliegende Erfindung geht davon aus, dass zur Untersuchung eines Patienten nur eine begrenzte Anzahl von Manövern einer Endoskopiekapsel benötigt wird, dass also die Auslegung einer Spulenanordnung hinsichtlich dieser tatsächlich benötigten Manöver zu optimieren ist. Insbesondere wird ein deutlich kostengünstigeres Design durch eine detaillierte Analyse der Anforderungen an die Spulenanordnung und eine Auslegung der Einzelspulen dergestalt erreicht, dass richtungsselektiv nur die Feldstärken und Feldgradienten realisierbar sein müssen, die bei Betrachtung der gewünschten Manöver der Endoskopiekapsel benötigt werden.

Bei der Auslegung der Spulenanordnung wird angenommen, dass in die Endoskopiekapsel ein Permanentmagnet fest eingebaut ist. Der Permanentmagnet ist also fest mit der Kapsel verbunden, so dass Bewegungen des Permanentmagneten, die durch die erfindungsgemäße Spulenanordnung ausgelöst werden, auf die Endoskopiekapsel übertragen werden. Das magnetisches Moment $\vec{m}$ des Permanentmagneten ist bevorzugt entweder in Richtung der Kapsellängsachse oder senkrecht zu dieser orientiert. Abhängig von diesen beiden Möglichkeiten der Permanentmagnetausrichtung ergeben sich leichte Unterschiede im erfindungsgemäßen magnetischen Führungssystem.

Die Optimierung hinsichtlich der tatsächlich benötigten Manöver der Endoskopiekapsel, die im Folgenden im Zusammenhang mit den Figuren am Beispiel einer Untersuchung der Speiseröhre (Ösophagus bzw. Esophagus), des Magens (Gaster) und des Zwölffingerdarms (Duodenum) beschrieben wird (sog. "EGD"-Untersuchung), führt zu einer Spulenanordnung zur Führung der Endoskopiekapsel mit nur noch zehn oder zwölf Einzelspulen. Dabei werden einige der zehn oder zwölf Einzelspulen paarweise, d.h. durch denselben Leistungsverstärker, betrieben. Dies schlägt sich vorteilhafterweise darin nieder, dass nur noch sechs, sieben oder acht Leistungsverstärker benötigt werden.

Die grundsätzliche Wirkungsweise der erfindungsgemäßen Spulenanordnung ist analog zur Wirkungsweise der in DE 103 40 925 B3 bzw. in WO 2006/092421 A1 beschriebenen Anordnung. Diesbezüglich und hinsichtlich theoretischer Grundlagen wird daher auf die DE 103 40 925 B3 und die WO 2006/092421 A1 verwiesen.

Vorteilhafterweise erlaubt die erfindungsgemäße Spulenanordnung, dass man gegenüber den in DE 103 40 925 B3 bzw. in WO 2006/092421 A1 beschriebenen Anordnungen durch ein geändertes Layout der Spulenanordnung mit weniger Einzelspulen und weniger Leistungsverstärkern dieselbe Anzahl magnetischer Freiheitsgrade (maximal acht) erzeugen kann. Werden aufgrund einer besonderen Anwendung bzw. Untersuchung einer oder mehrere dieser Freiheitsgrade nicht benötigt, kommt man mit weniger Leistungsverstärkern oder ggf. zusätzlich auch mit weniger Spulen aus.

Weitere Vorteile, Merkmale und Einzelheiten der Erfindung ergeben sich aus dem im Folgenden beschriebenen Ausführungsbeispiel sowie anhand der Zeichnungen.

Dabei zeigt:

Figur 1    eine schematische Ansicht eines Ausführungsbeispiels der erfindungsgemäßen Spulenanordnung,

Figur 2    eine schematische Ansicht verschiedener Spulenpaare der Spulenanordnung mit Kennzeichnung des Stromflusses durch die Spulenpaare,

Figur 3a    eine schematische Ansicht eines weiteren Ausführungsbeispiels der erfindungsgemäßen Spulenanordnung,

Figur 3b    eine perspektivische Ansicht des Ausführungsbeispiels der Figur 3a,

Figur 3c    eine alternative Ausführung des Ausführungsbeispiels der Figur 3a,

Figur 4a    eine Flächenspule,

Figur 4b    eine in Teilspulen aufgetrennte Flächenspule,

Figur 5a    einen Querschnitt durch eine erfindungsgemäße Spulenanordnung,

Figur 5b    eine Sattelspule in perspektivischer Darstellung,

Figur 6    eine Ringspule in perspektivischer Darstellung,

Figur 7    eine schematische Ansicht eines weiteren Ausführungsbeispiels der erfindungsgemäßen Spulenanordnung und

Figur 8    Zylinderquerschnitte in schematischer Ansicht.

Erstes Ausführungsbeispiel

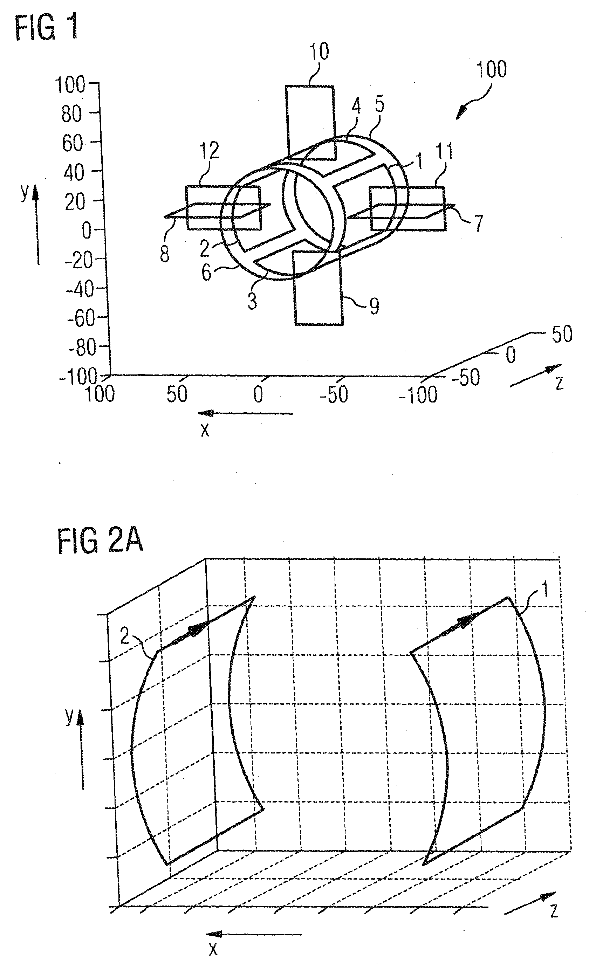

Die Figur 1 zeigt eine erfindungsgemäße Spulenanordnung 100, die vorgesehen ist zur Führung bzw. Navigation eines magnetischen Elements, insbesondere einer Endoskopiekapsel 200, im Inneren eines Patienten (nicht dargestellt), die einen Permanentmagneten mit einem magnetischen Dipolmoment $\vec{m}$ in Richtung der Kapsellängsachse enthält (nicht dargestellt). Der Permanentmagnet ist fest mit der Endoskopiekapsel 200 verbunden, so dass Bewegungen des Permanentmagneten, die durch die Spulenanordnung 100 erzeugt werden, direkt auf die Endoskopiekapsel 200 übertragen werden. Insbesondere kann die Spulenanordnung 100 Magnetfeldkomponenten $B_x$, $B_y$, $B_z$ sowie bestimmte Gradientenfelder ($B_x / \partial x$ etc.) der Gradientenmatrix $\underline{G}$ erzeugen, die in einem kartesischen Koordinatensystem x, y, z wie in der Figur 1 dargestellt definiert sind. Die Längsachse der Spulenanordnung 100 entspricht der z-Achse des Koordinatensystems. Diese Definition des Koordinatensystems gilt in gleicher Form auch für die weiteren Ausführungsbeispiele.

Die Spulenanordnung 100 des ersten Ausführungsbeispiels beinhaltet im Unterschied zu den in DE 103 40 925 B3 und in WO 2006/092421 A1 beschriebenen Spulenanordnungen mit vierzehn Einzelspulen nur noch zwölf Einzelspulen 1-12, umfassend eine erste 1 bis zwölfte Spule 12.

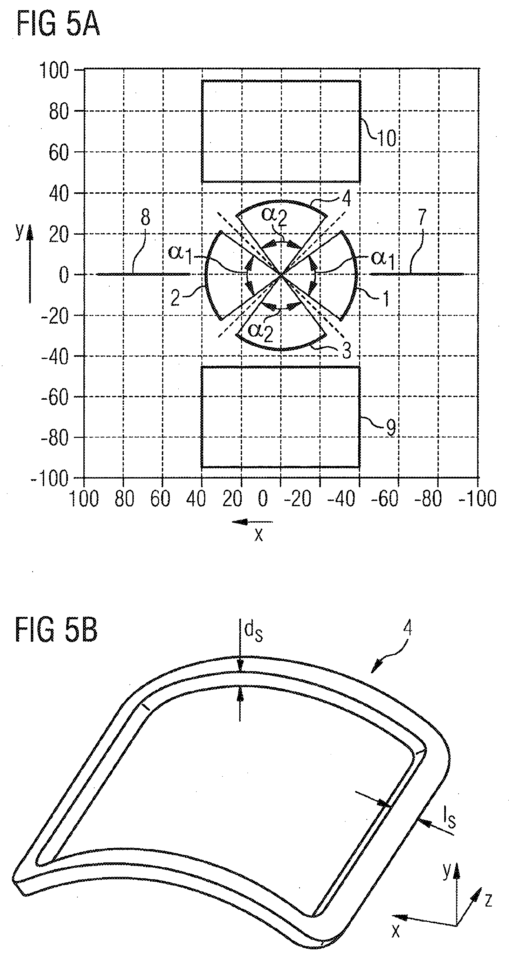

Vier der zwölf Einzelspulen 1-12, insbesondere die erste 1 bis vierte Spule 4, der Spulenanordnung 100 sind als identisch geformte Sattelspulen 1-4 ausgebildet und umfassen einen Arbeitsraum A, in dem der Patient positioniert wird. Die Sattelspulen 1-4 bilden eine (gedachte) Mantelfläche eines Zylinders mit kreisförmigem Querschnitt und erstrecken sich entlang des Umfangs der Mantelfläche jeweils über denselben Raumwinkel $\alpha_1 = \alpha_2$ (vgl. Figur 5a). Das Zentrum des Zylinders befindet sich im Ursprung des kartesischen Koordinatensystems, die Längs- bzw. Symmetrieachse des Zylinders ist in z-Richtung orientiert. Die Sattelspulen 1-4 sind in Umfangsrichtung der Mantelfläche gesehen vorteilhaft gegenseitig beabstandet angeordnet, d.h. zwischen ihren stirnseitigen Abschnitten und somit zwischen ihren in z-Richtung verlaufenden Längsseiten befindet sich jeweils ein Zwischenraum, der eine thermische Ausdehnung der Sattelspulen zulässt. Die ersten Sattelspulen 1, 2 bilden ein erstes Spulenpaar 1, 2 und die zweiten Sattelspulen 3, 4 bilden ein zweites Spulenpaar 3, 4.

Die fünfte 5 und die sechste Spule 6 bilden den Abschluss des Zylinders in z-Richtung und sind als Ringspulen ausgebildet, d.h. die Einzelspulen 5, 6 sind ebene, kreisförmige Spulen und weisen einen rechteckigen Querschnitt auf. Die Ringspulen 5, 6 liegen jeweils in einer Ebene parallel zur x-y-Ebene und bilden ein drittes Spulenpaar 5, 6. In z-Richtung gesehen befinden sich die Ringspulen 5, 6 an Positionen $+ z_r$ und $- z_r$, bspw. mit $z_r = 400mm$. Die Mittelpunkte der Ringspulen 5, 6 liegen auf der z-Achse, die Innendurchmesser der Ringspulen 5, 6 entsprechen dem Innendurchmesser $d_M$ der durch die Sattelspulen 1-4 gebildeten Mantelfläche, wobei bspw. $d_M = 620mm$ gelten kann. Die Länge der Mantelfläche in z-Richtung wird definiert durch die Länge der Sattelspulen 1-4 in z-Richtung. Gemäß der Figur 5b kann eine Sattelspule bspw. 700mm lang sein.

Die Sattelspulen 1-4 und/oder die Ringspulen 5, 6 können alternativ als Flächenspulen gestaltet sein. Die oben beschriebene Lage der jeweiligen Spulen im Koordinatensystem kann dabei unverändert bleiben. Flächenspulen zeichnen sich dadurch aus, dass sie bspw. im Unterschied zu Sattelspulen als ebene Spulen ausgebildet sind und vorzugsweise einen rechteckigen Querschnitt aufweisen.

Weitere sechs Einzelspulen 7-12, insbesondere die siebte 7 bis zwölfte Spule 12, sind flügelartig angeordnet und als rechteckige Flächenspulen 7-12 ausgebildet, wobei die Seiten des jeweiligen Rechtecks parallel zu den entsprechenden Achsen des Koordinatensystems ausgerichtet sind. Dabei bezeichnet der Begriff "flügelartig", dass die jeweilige Einzelspule in der x-y-Ebene, in der x-z-Ebene oder in der y-z-Ebene und gleichzeitig radial außerhalb der Mantelfläche des durch die Sattelspulen 1-4 gebildeten Zylinders angeordnet ist, wie in der Figur 1 ersichtlich.

Grundsätzlich gilt, dass die Einzelspulen 1-12 möglichst nah am Arbeitsraum A platziert werden sollten. Speziell die Flächenspulen 7-12 werden derart dimensioniert, dass sie vom Koordinatenursprung aus gesehen deutlich flacher als breit sind.

Die ersten Flächenspulen 7, 8 befinden sich in der x-z-Ebene, d.h. in y-Richtung an Positionen y = 0, und bilden ein viertes Spulenpaar 7, 8. Die radial innen liegenden leitenden Abschnitte der ersten Flächenspulen 7, 8 befinden sich in x-Richtung an Positionen $+ r_f$ und $- r_f$, wobei bspw. $2r_f = 840mm$ gelten kann. In z-Richtung sind die ersten Flächenspulen 7, 8 derart positioniert, dass ihre geometrischen Mittelpunkte bei z = 0 liegen.

Die zweiten Flächenspulen 9, 10 befinden sich in der x-y-Ebene, d.h. in z-Richtung an Positionen z = 0, und bilden ein fünftes Spulenpaar 9, 10. Die radial innen liegenden leitenden Abschnitte der zweiten Flächenspulen 9, 10 befinden sich in y-Richtung an Positionen $+ r_f$ und $- r_f$. In x-Richtung sind die zweiten Flächenspulen 9, 10 derart positioniert, dass ihre geometrischen Mittelpunkte bei x = 0 liegen.

Weiterhin sind dritte Flächenspulen 11, 12 vorgesehen, die ein sechstes Spulenpaar 11, 12 bilden. Die Flächenspulen 11, 12 befinden sich in der x-y-Ebene, d.h. in z-Richtung an Positionen z = 0, und sind gekreuzt zu den ersten Flächenspulen 7, 8 und innerhalb der ersten Flächenspulen 7, 8 angeordnet. Die radial innen liegenden leitenden Abschnitte der zusätzlichen Flächenspulen 11, 12 befinden sich in x-Richtung an Positionen $+ r_f$ und $- r_f$. In y-Richtung sind die zusätzlichen Flächenspulen 11, 12 derart positioniert, dass ihre geometrischen Mittelpunkte bei y = 0 liegen.

Ein Spulenpaar, d.h. ein Sattelspulenpaar, ein Ringspulenpaar bzw. ein Flächenspulenpaar, zeichnet sich dadurch aus, dass die beiden dem Spulenpaar zugehörigen Einzelspulen bezüglich des Ursprungs des kartesischen Koordinatensystems (x, y, z) punktsymmetrisch zueinander sind. Die die geometrischen Mittelpunkte der Spulen eines Spulenpaars verbindende Linie geht durch den Ursprung des Koordinatensystems. Zusätzlich gilt insbesondere für ein Sattelspulenpaar und ein Ringspulenpaar, dass die die geometrischen Mittelpunkte der Spulen dieser Spulenpaare jeweils verbindende Linie senkrecht auf der Querschnittsfläche der Spulen steht. Unter der Querschnittsfläche einer Sattelspule wird dabei die Fläche verstanden, die durch die beiden in z-Richtung verlaufenden leitenden Abschnitte definiert wird. Bspw. die Querschnittsfläche der Sattelspule 4 läge demnach gemäß der Figur 5b in einer Ebene parallel zur y-z-Ebene. Ggf. können die beiden einem Spulenpaar zugehörigen Einzelspulen durch einen gemeinsamen Leistungsverstärker bestromt werden, anstatt die beiden Einzelspulen jeweils durch einen separaten Leistungsverstärker mit Strom zu versorgen.

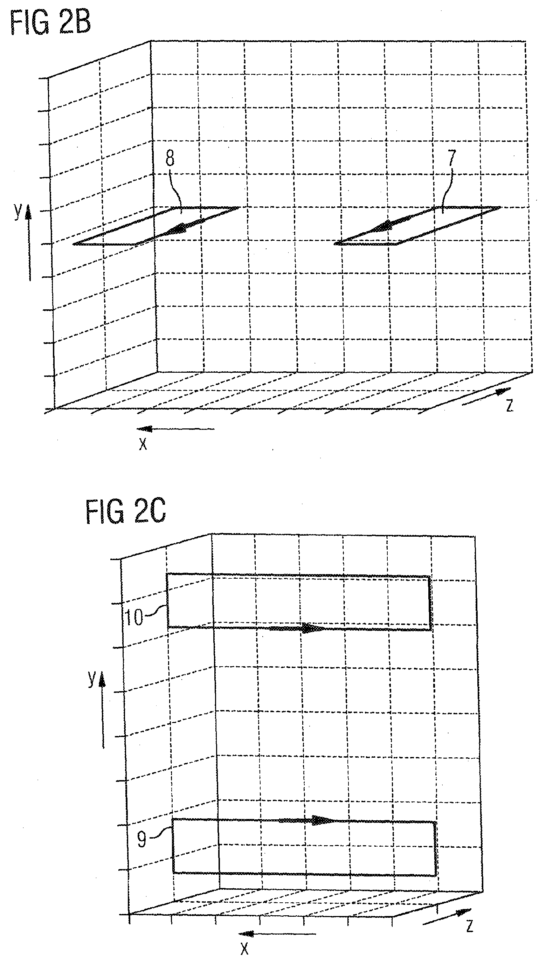

Die Figuren 2a-2c zeigen den Stromfluss durch die Spulenpaare 1 und 2 (Figur 2a), 7 und 8 (Figur 2b) sowie 9 und 10 (Figur 2c), wobei die Richtung des Stroms durch Pfeile symbolisiert wird. Diese drei Spulenpaare werden jeweils von nur einem Leistungsverstärker betrieben. Das Spulenpaar 1, 2 erzeugt hiermit die Komponente $B_x$ des Magnetfeldes $\vec{B}$. Das Spulenpaar 7, 8 wird zur Erzeugung des Gradientenfeldes $\partial B_y / \partial x$ ($=\partial B_x / \partial y$) eingesetzt und das Spulenpaar 9, 10 erzeugt das Gradientenfeld $\partial B_z / \partial y$.

Die Spulen 3 bis 6 werden im Unterschied zu den oben, im Zusammenhang mit der Figur 2 beschriebenen Spulenpaaren individuell von separaten Leistungsverstärkern betrieben und erzeugen zum Einen die Komponenten $B_y$ (Einzelspulen 3, 4) und $B_z$ (Einzelspulen 5, 6) des Magnetfeldes $\vec{B}$. Zum Anderen werden die Gradientenfelder $\partial B_y / \partial y$ und $\partial B_z / \partial z$ erzeugt, indem die Einzelspulen 3 und 4 zur Erzeugung des Gradientenfeldes $\partial B_y / \partial y$ mit unterschiedlichen Strömen versorgt werden. Dementsprechend wird das Gradientenfeld $\partial B_z / \partial z$ durch unterschiedliche Ströme in den Einzelspulen 5 und 6 aufgebaut.

Die Flächenspulen 11, 12 werden durch einen gemeinsamen Leistungsverstärker betrieben und erzeugen ein Gradientenfeld $\partial B_z / \partial x$.

Mit der Spulenanordnung 100 des ersten Ausführungsbeispiels ist es demnach möglich, mit nur zwölf Einzelspulen und acht Leistungsverstärkern sämtliche acht magnetischen Freiheitsgrade zu realisieren, d.h. die Magnetfeldkomponenten $B_x, B_y, B_z$ sowie die fünf Gradientenfelder $\partial B_y / \partial y, \partial B_z / \partial z, \partial B_x / \partial y, \partial B_z / \partial x, \partial B_z / \partial y$ der Gradientenmatrix $\underline{G}$.

Die Spulenanordnung 100 des ersten Ausführungsbeispiels ist starr ausgeführt, d.h. die Einzelspulen 1-12 sind mechanisch nicht beweglich und es sind keine Permanentmagnete vorgesehen. Gleiches gilt für die Spulenanordnungen der weiteren Ausführungsbeispiele.

Vor der Beschreibung weiterer Ausführungsbeispiele wird im Folgenden die weitere Optimierung hinsichtlich der tatsächlich benötigten Manöver der Endoskopiekapsel beschrieben.

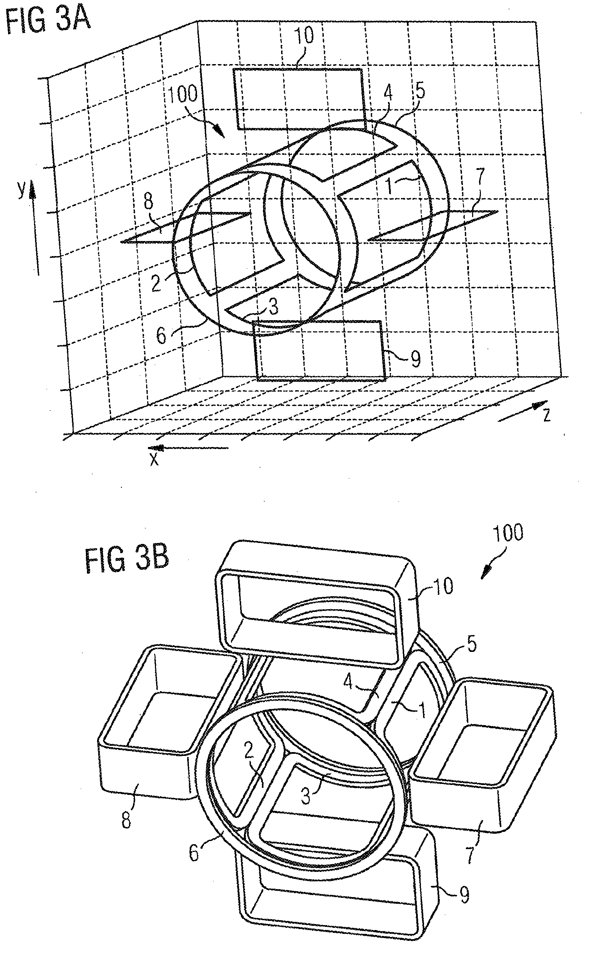

Zur vollständigen Untersuchung der Speiseröhre (Ösophagus bzw. Esophagus), des Magens (Gaster) und des Zwölffingerdarms (Duodenum) des Patienten ist eine eingeschränkte Anzahl von Manövern M1-M5 der Endoskopiekapsel 200 notwendig. Dementsprechend kann die Spulenanordnung 100 für die spezielle Anwendung gemäß den Figuren 3a/b, 8 und 9 ausgelegt werden. Der Patient befindet sich üblicherweise auf dem Rücken oder auf dem Bauch mit der Körperlängsachse in z-Richtung liegend innerhalb der Spulenanordnung 100.

M1: Zur Untersuchung der Speiseröhre muss die Endoskopiekapsel 200 lediglich in z-Richtung bewegt werden, da auch die Speiseröhre in guter Näherung in z-Richtung orientiert ist. Es muss demnach eine Kraft in z-Richtung erzeugt werden. Dementsprechend muss die Spulenanordnung ein Gradientenfeld $\partial B_z / \partial z$ aufbauen.

M2: Für die Navigation der Kapsel 200 im Magen wird vorausgesetzt, dass er zum Teil mit Flüssigkeit gefüllt ist. Der Patient muss hierfür vor und während der Untersuchung Wasser zu sich nehmen. Um die in y-Richtung ausgerichtete Endoskopiekapsel mit in Kapsellängsrichtung magnetisiertem Permanentmagneten an der Wasseroberfläche, d.h. in x- und/oder z-Richtung, zu manövrieren, sind Gradientenfelder $\partial B_y / \partial x$ (= $\partial B_x / \partial y$) und $\partial B_y / \partial z$ (= $\partial B_z / \partial y$) notwendig. Die in Klammern gesetzten Ausdrücke ergeben sich zwangsläufig aufgrund der Maxwell-Gleichung rot $\vec{B}$ = 0.

M3: Um im Magen die Position der Endoskopiekapsel 200 an der Wasseroberfläche zu halten, muss eine magnetische Kraft erzeugt werden, die der Gewichtskraft entgegenwirkt. Dabei wird wieder davon ausgegangen, dass das magnetische Moment $\vec{m}$ der Endoskopiekapsel 200 in y-Richtung orientiert ist. Es muss demnach ein Gradientenfeld $\partial B_y / \partial y$ erzeugt werden. Dieses kann darüber hinaus verwendet werden, um die Endoskopiekapsel 200 unter- und wieder auftauchen zu lassen.

M4: Bspw. zur Aufnahme von Bildern der Mageninnenwand kann es nötig sein, die Endoskopiekapsel 200 in eine bestimmte, beliebige Richtung $\vec{r}$ zu drehen. Hierzu muss die Spulenanordnung 100 ein magnetisches Feld $\vec{B}$ parallel zur gewünschten Richtung $\vec{r}$ aufbauen. Dementsprechend muss es möglich sein, sämtliche Komponenten $B_x, B_y, B_z$ des magnetischen Feldes $\vec{B}$ zu erzeugen.

M5: Um die Endoskopiekapsel 200, die weiterhin im Wesentlichen in y-Richtung orientiert ist, durch den Magenausgang zu führen, ist wieder ein Gradientenfeld $\partial B_y / \partial y$ notwendig.

Zweites Ausführungsbeispiel

In einem zweiten Ausführungsbeispiel wird die in den Figuren 3a/b dargestellte Spulenanordnung 100 dazu verwendet, die Endoskopiekapsel 200 im Magen des Patienten zu navigieren. Hierzu kann angenommen werden, dass die Endoskopiekapsel 200 über den normalen Schluckvorgang in den Magen befördert wurde. Zum Manövrieren der Kapsel 200 im Magen sind die oben aufgeführten Manöver M2 bis M4 notwendig. Dementsprechend muss die Spulenanordnung 100 des ersten Ausführungsbeispiels ein Magnetfeld $\vec{B}$ in beliebiger Richtung sowie die Gradientenfelder $\partial B_y / \partial y, \partial B_y / \partial x$ und $\partial B_z / \partial y$ erzeugen. Im Vergleich zum ersten Ausführungsbeispiel ist es demnach nicht notwendig,

ein Gradientenfeld $\partial B_z / \partial x$ zu erzeugen. Auf die dritten Flächenspulen 11, 12 kann daher verzichtet werden, so dass im zweiten Ausführungsbeispiel nur zehn Einzelspulen 1-10, d.h. die erste 1 bis zehnte Spule 10, benötigt werden.

Zur Erzeugung der genannten Magnetfeldkomponenten und Gradientenfelder im zweiten Ausführungsbeispiel werden darüber hinaus nur sechs Leistungsverstärker benötigt: Die aus den Einzelspulen 1 und 2, 5 und 6, 7 und 8 sowie 9 und 10 bestehenden vier Spulenpaare werden jeweils über einen gemeinsamen Leistungsverstärker versorgt und erzeugen die Magnetfeldkomponenten bzw. Gradientenfelder $B_x$, $B_z$, $\partial B_y / \partial x$ sowie $\partial B_z / \partial y$. Die Einzelspulen 3 und 4 werden zur Erzeugung der Magnetfeldkomponente $B_y$ und des Gradientenfeldes $\partial B_y / \partial y$ mit unterschiedlichen Strömen und deswegen mit zwei einzelnen Leistungsverstärkern betrieben.

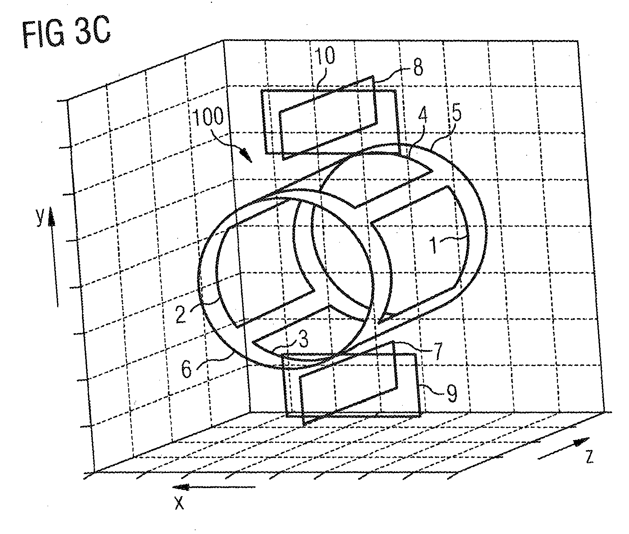

Da die Gradientenmatrix $\underline{G}$ wie oben beschrieben symmetrisch ist, gilt $\partial B_y / \partial x = \partial B_x / \partial y$. Anstelle der in der Figur 3a dargestellten Positionierung der Einzelspulen 7, 8 zur Erzeugung des Gradientenfeldes $\partial B_y / \partial x$ können die Einzelspulen 7, 8 wie in der Figur 3c gezeigt zur Erzeugung eines Gradientenfeldes $\partial B_x / \partial y$ gekreuzt zu den 9, 10 angeordnet werden. Das Spulenpaar 7, 8 wird dabei weiterhin über einen gemeinsamen Leistungsverstärker versorgt. Die Flächenspulen 7, 8 befinden sich nun in der y-z-Ebene, d.h. in x-Richtung an Positionen x = 0. In z-Richtung sind die ersten Flächenspulen 7, 8 derart positioniert, dass ihre geometrischen Mittelpunkte bei z = 0 liegen.

Drittes Ausführungsbeispiel

Die Spulenanordnung 100 im dritten Ausführungsbeispiel ist vorgesehen zur Führung der parallel zu ihrer Längsachse magnetisierten Endoskopiekapsel 200 durch die Speiseröhre (Ösophagus bzw. Esophagus), den Magen (Gaster) und den Zwölffingerdarm (Duodenum) des Patienten, d.h. für eine vollständige EGD-Untersuchung. Dementsprechend sind im dritten Ausführungsbeispiel sämtliche der oben angeführten Manöver M1 bis M5 zu realisieren.

Die Spulenanordnung 100 muss demnach derart ausgebildet sein, dass ein Magnetfeld $\vec{B}$ in beliebiger Richtung sowie Gradientenfelder $\partial By / \partial y$, $\partial B_z / \partial z$, $\partial B_y / \partial x$ und $\partial B_z / \partial y$ erzeugt werden können. Hierzu sind wie im zweiten Ausführungsbeispiel zehn Einzelspulen 1-10 notwendig. Im Unterschied zum zweiten Ausführungsbeispiel werden jedoch sieben Leistungsverstärker benötigt: Die drei Spulenpaare bestehend aus der ersten 1 und der zweiten Spule 2, der siebten 7 und der achten Spule 8 sowie der neunten 9 und der zehnten Spule 10 werden jeweils über einen Leistungsverstärker betrieben und erzeugen die Felder bzw. Gradientenfelder $\partial_x$, $\partial B_y / \partial x$ sowie $\partial B_z / \partial y$. Die dritte 3 und die vierte Spule 4 werden zur Erzeugung der Magnetfeldkomponente $B_y$ und des Gradientenfeldes $\partial B_y / \partial y$ mit unterschiedlichen Strömen und deswegen mit zwei einzelnen Leistungsverstärkern betrieben. Genauso werden die Spulen 5 und 6 zur Erzeugung von $B_z$ und $\partial B_z / \partial z$ mit zwei einzelnen Leistungsverstärkern betrieben.

Viertes Ausführungsbeispiel

In einem vierten Ausführungsbeispiel ist das magnetische Moment des Permanentmagneten der Endoskopiekapsel 200 senkrecht zur Längsachse der Endoskopiekapsel 200 ausgerichtet.

Um wie im zweiten Ausführungsbeispiel eine Untersuchung des Magens durchzuführen, müssen die Manöver M2' bis M4' ausführbar sein, die an die oben beschriebenen Manövern M2 bis M4 angelehnt sind:

M2': Bei Navigation der in vertikaler Richtung ausgerichteten Kapsel 200 an der Wasseroberfläche im Magen liegt das magnetische Moment $\vec{m}$ der Endoskopiekapsel in der x-z-Ebene. Um die Endoskopiekapsel in x- und/oder z-Richtung zu manövrieren, werden Gradientenfelder $\partial B_x / \partial x$ und $\partial B_z / \partial z$ benötigt.

M3': Um im Magen die Position der Endoskopiekapsel 200 an der Wasseroberfläche zu halten oder um die Kapsel 200 in vertikaler Richtung zu bewegen, müssen Gradientenfelder $\partial B_y / \partial x$ und $\partial B_z / \partial y$ erzeugt werden.

M4': Zur Drehung der Endoskopiekapsel 200 in eine bestimmte, beliebige Richtung $\vec{r}$ muss die Spulenanordnung 100 ein magnetisches Feld $\vec{B}$ parallel zur gewünschten Richtung r aufbauen.

Zusammenfassend werden demnach zur Magenuntersuchung mit einer diametral magnetisierten Kapsel 200 die Magnetfeldkomponenten $B_x$, $B_y$, $B_z$ sowie die Gradientenfelder $\partial B_x / \partial x$, $\partial B_z / \partial z$, $\partial B_y / \partial x$ und $\partial B_z / \partial y$ benötigt.

Zur Erzeugung dieser Felder kann wieder eine Spulenanordnung mit zehn Einzelspulen 1-10 wie in den Figuren 3a/b dargestellt verwendet werden, wobei die aus den Einzelspulen 3 und 4, 7 und 8 sowie 9 und 10 bestehenden drei Spulenpaare jeweils über einen gemeinsamen Leistungsverstärker versorgt werden, um die Felder bzw. Gradientenfelder $B_y$, $\partial B_y / \partial x$ sowie $\partial B_z / \partial y$ zu erzeugen. Die Einzelspulen 1 und 2 werden zur Erzeugung der Magnetfeldkomponente $B_x$ und des Gradientenfeldes $\partial B_x / \partial x$ mit unterschiedlichen Strömen und deswegen mit zwei einzelnen Leistungsverstärkern betrieben. Genauso werden die Einzelspulen 5 und 6 zur Erzeugung von $B_z$ und $\partial B_z / \partial z$ mit zwei einzelnen Leistungsverstärkern betrieben. Es werden demnach sieben Leistungsverstärker benötigt.

Fünftes Ausführungsbeispiel

Die Spulenanordnung 100 eines fünften Ausführungsbeispiels ist identisch zur Spulenanordnung 100 des ersten Ausführungsbeispiels und umfasst zwölf Einzelspulen 1-12 (Figur 1) und acht Leistungsverstärker. Zur Verschaltung der Einzelspulen 1-12 und der Leistungsverstärker wird auf das erste Ausführungsbeispiel verwiesen. Wie im Zusammenhang mit dem ersten Ausführungsbeispiel beschrieben, ist es mit der zwölf Einzelspulen 1-12 umfassenden Spulenanordnung 100 gemäß Figur 1 möglich, sämtliche acht magnetischen Freiheitsgrade zu erzeugen. Demnach können auch sämtliche im Zusammenhang mit der diametral magnetisierten Endoskopiekapsel 200 benötigten Manöver durchgeführt werden. Die Spulenanordnung 100 des ersten und des fünften Ausführungsbeispiels ist demnach universell für Endoskopiekapseln 200 einsetzbar, deren magnetisches Moment $\vec{m}$ parallel oder senkrecht zur Kapsellängsachse ausgerichtet ist. Für eine diametral magnetisierte Endoskopiekapsel 200 ergeben sich gegenüber dem ersten Ausführungsbeispiel aufgrund anderer zu erzeugender Kräfte und Drehmomente allerdings Unterschiede in der Auslegung der Spulenanordnung 100 bezüglich der Bestromung der Einzelspulen und der Auslegung der Einzelspulen bspw. hinsichtlich der Amperewindungszahl.

Weitere Ausführungsformen

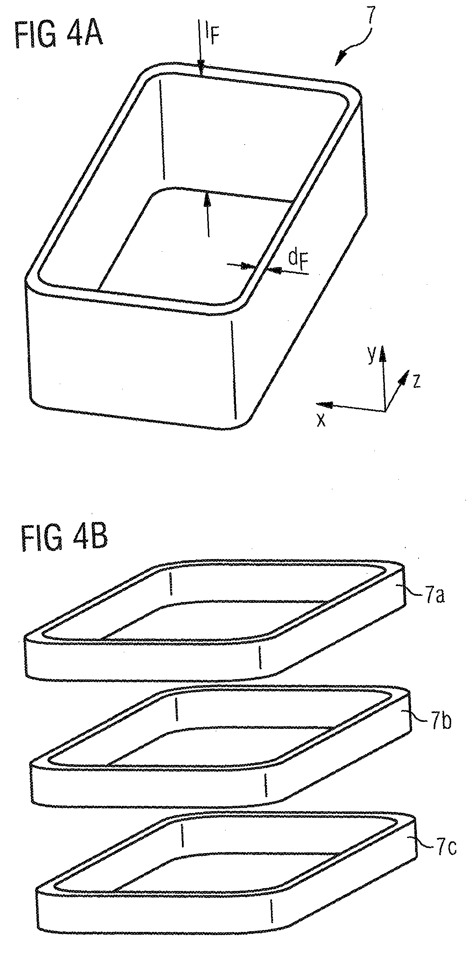

Die Flächenspulen 7-12 können derart dimensioniert sein, dass sie vom Koordinatenursprung aus gesehen deutlich flacher als breit sind. Dabei bezieht sich das Merkmal "flach" am Beispiel der Flächenspule 7 in der Figur 4a auf die Dicke $d_F$ des leitenden Abschnitts, d.h. in x-Richtung gesehen auf die Ausdehnung des innen (und des außen) liegenden leitenden Abschnitts. Das Merkmal "breit" beschreibt die Ausdehnung $l_F$ des leitenden Abschnitts in y-Richtung. Für die Flächenspule 7 kann demnach so ausgelegt sein, dass gilt $d_F < l_F$. Die Flächenspule 8 kann identisch dimensioniert werden. Die übrigen Flächenspulen 9 und 10 bzw. 11 und 12 können genauso ausgebildet sein. Dabei müssen die für die verschiedenen Spulenpaare 7 und 8, 9 und 10 sowie ggf. 11 und 12 gewählten Bemaßungen $d_F$, $l_F$ nicht übereinstimmen.

Die Flächenspulen 7-12 können jeweils in mehrere einzelne Teilspulen aufgetrennt sein. Wieder am Beispiel der Flächenspule 7 der Figur 4a kann die in der x-z-Ebene liegende Flächenspule 7 in y-Richtung, d.h. in Richtung des auf der Querschnittsfläche der Spule 7 stehenden Normalenvektors, übereinander liegende Teilspulen 7a, 7b und 7c aufgetrennt werden, wie in der Figur 4b dargestellt. Die Flächenspule 8 wäre dementsprechend ebenfalls in Teilspulen 8a, 8b und 8c aufgetrennt (nicht dargestellt). Vorzugsweise stimmen die Bemaßungen der Teilspulen 7a und 8a, 7b und 8b sowie 7c und 8c überein. Hierdurch wird vorteilhafterweise eine Homogenisierung des durch die Flächenspulen 7 und 8 erzeugbaren Feldgradienten $\partial B_y / \partial x$ erreicht. Dabei kann zwischen zwei übereinander liegenden Spulen ein Zwischenraum in der Größenordnung von 1cm liegen. Dementsprechend können auch die übrigen Flächenspulen 9-12 in mehrere Teilspulen aufgetrennt werden mit der entsprechenden Homogenisierung ihrer Gradientenfelder. Die Auftrennung in mehr oder weniger Teilspulen als in der Figur 4b dargestellt ist ebenfalls denkbar.

Eine weitere Ausführungsform der Spulenanordnung 100 gemäß der Figur 5a bezieht sich lediglich auf die Sattelspulen 1-4. Die spezielle Ausgestaltung der Sattelspulen 1-4 kann die im Rahmen der im Vorfeld beschriebenen Ausführungsbeispiele enthaltenen Sattelspulen 1-4 ersetzen. Die Sattelspulen 1-4 können hinsichtlich des Raumwinkels $\alpha$ entlang des Umfangs der Mantelfläche unterschiedlich ausgebildet sein. Die erste Spule 1 und die zweite Spule 2 erstrecken sich entlang des Umfangs der Mantelfläche jeweils über einen ersten Raumwinkel $\alpha_1$, während sich die dritte Spule 3 und die vierte Spule 4 jeweils über einen zweiten Raumwinkel $\alpha_2$ erstrecken. Um zum Einen das magnetische Feld $B_y$ in y-Richtung und zum Anderen auch das Gradientenfeld $\partial B_y / \partial y$ zu verstärken, werden die Raumwinkel $\alpha_2$ der Sattelspulen 3 und 4 auf $\alpha_2 > 90°$ vergrößert. Vorzugsweise liegen die Raumwinkel jedoch im Bereich $90° < \alpha_2 < 110°$. Der erste Raumwinkel $\alpha_1$ der Sattelspulen 1 und 2 ist dann entsprechend zu verkleinern.

In der Figur 5b ist eine bevorzugte Ausführungsform der Sattelspule 4 gezeigt. Die übrigen Sattelspulen können dementsprechend dimensioniert sein. Die Sattelspule 4 ist flach ausgebildet, d.h. die Ausdehnung $d_s$ des leitenden Abschnitts in radialer Richtung kann kleiner sein als die Querausdehnung $1_s$ des leitenden Abschnitts in z.B. z-Richtung.

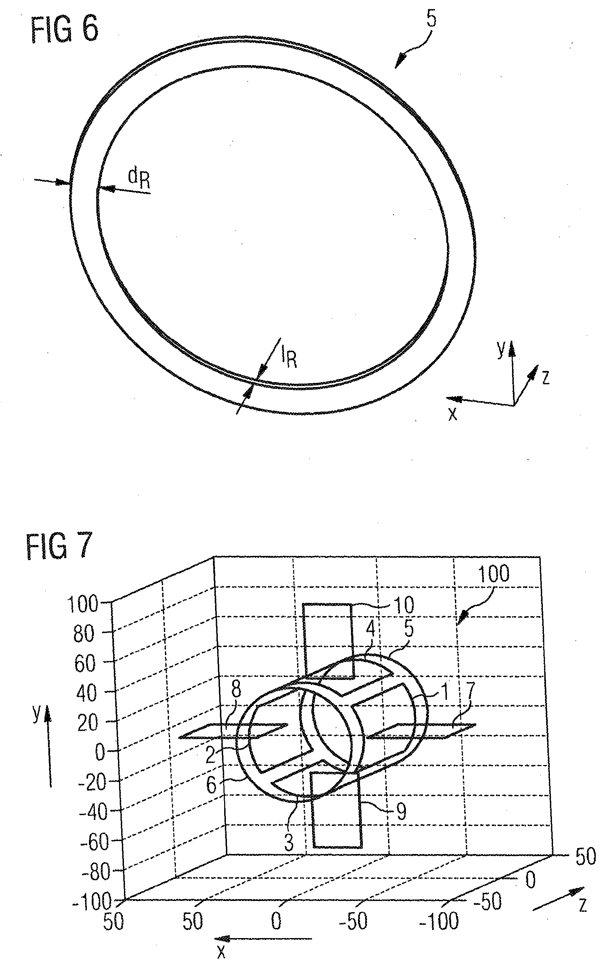

Die Figur 6 zeigt eine Ausführung der Ringspule 5, die in z-Richtung gesehen flach ausgeführt ist. Dies bedeutet, dass die Ausdehnung $l_R$ in z-Richtung kleiner sein kann als die Stärke $d_R$ des Ringes, d.h. als die Ausdehnung $d_R$ der Ringspule 5 in radialer Richtung. Die Ringspule 6 kann identisch ausgebildet sein.

In einer weiteren Ausführungsform, die in der Figur 7 skizziert ist, werden die Flächenspulen 7, 8 und/oder 9, 10 räumlich näher an den Arbeitsraum A herangeführt. Dies hat den Vorteil, dass die den Nebendiagonalelementen der Gradientenmatrix G entsprechenden Gradientenfelder, im vorliegenden Fall insbesondere die Gradientenfelder $\partial B_y / \partial x$ und/oder $\partial B_z / \partial y$, verstärkt werden, ohne dass leistungsfähigere Elektronik benötigt wird. Hierzu werden die Flächenspulen 7, 8 und/oder 9, 10 räumlich z.T. in die Sattelspulen integriert, so dass zumindest die radial innen liegenden leitenden Abschnitte der Flächenspulen 7, 8 und/oder 9, 10 näher am Arbeitsraum A liegen. Im Unterschied zu den oben beschriebenen Ausführungsbeispielen befinden sich die Flächenspulen 7, 8 und/oder 9, 10 nun nicht mehr vollständig radial außerhalb der Mantelfläche des durch die Sattelspulen 1-4 gebildeten Zylinders. Die Flächenspulen 7, 8 und/oder 9, 10 müssen hierfür entsprechend der Dimensionierung der Sattelspulen in z-Richtung verkürzt werden, was den zusätzlichen Vorteil mit sich bringen kann, dass das Gewicht der Flächenspulen 7, 8 und/oder 9, 10 reduziert wird.

Die oben beschriebenen Ausführungsbeispiele unterscheiden sich neben der Anzahl der zu verwendenden Einzelspulen, der Kapselmagnetisierungsrichtung und dem Betreiben der Einzelspulen durch Leistungsverstärker als Spulenpaar oder einzeln auch darin, dass die konkreten Ausführungsformen hinsichtlich der Bemaßungen und des Leistungsbedarfs der Einzelspulen 1-10 bzw. 1-12 variieren können.

Bei den im Zusammenhang mit den Figuren 1 und 3c gezeigten Ausführungsbeispielen mit gekreuzten Spulen kann jeweils eine der gekreuzten Spulen innerhalb der anderen Spule liegen, d.h. die innen liegende Spule muss entsprechend kleiner gestaltet werden als die außen liegende, umgebende Spule. Alternativ können die die leitenden Abschnitte überschneiden, d.h. dass sich am Schnittpunkt der Spulen die Leiterbahnen der beiden Spulen abwechseln.

Es wird darauf hingewiesen, dass die obige Definition des Koordinatensystems x,y,z willkürlich ist, insbesondere betreffend die Ausrichtung der x- und der y-Achse. Eine Drehung der Spulenanordnung 100 nach einem der oben beschriebenen Ausführungsbeispiele um einen beliebigen Drehwinkel um die Längsachse der Spulenanordnung, d.h. um die z-Achse des Koordinatensystems, wird nicht weiter beschrieben, ist aber ebenfalls Gegenstand der vorliegenden Erfindung. Dies betrifft insbesondere das erste und das fünfte Ausführungsbeispiel sowie deren weitere Ausführungen, da mit diesen sämtliche magnetischen Freiheitsgrade erzeugbar sind. Eine Drehung der Spulenanordnung 100 um die z-Achse um bspw. 90° bringt eine Spulenanordnung 100' hervor, mit der ebenfalls sämtliche magnetischen Freiheitsgrade erzeugt werden können. Die Spulenanordnung 100' wirkt demnach genauso wie die Spulenanordnung 100.

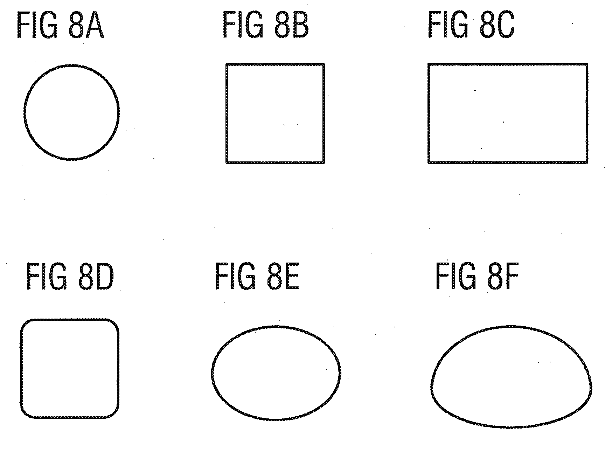

Alle beschriebenen Ausführungsbeispiele der Spulenanordnungen 100 enthalten vier Sattelspulen 1-4 und zwei Ringspulen 5, 6, die alle auf der Mantelfläche eines Zylinders mit kreisförmigem, in der Figur 8a schematisch dargestelltem Querschnitt liegen bzw. die Mantelfläche eines Kreiszylinders beschreiben. Alternativ kann dieser 6-Spulen-Zylinder aber auch einen quadratischen oder rechteckigen Querschnitt aufweisen (Figuren 8b, 8c). Statt der vier Sattelspulen werden dann vier Flachspulen und statt der zwei Ringspulen werden dann zwei Rechteckspulen verwendet. Weiterhin kann der Querschnitt des Zylinders auch quadratisch oder rechteckig mit abgerundeten Ecken sein, wie in der Figur 8d angedeutet. Auch ein elliptischer (Figur 8e) oder ein unsymmetrischer Querschnitt (Figur 8f), bspw. ein abgeplatteter Kreis, sind denkbar. Der Querschnitt des Zylinders wird durch die Formgebung der Einzelspulen 1-6 realisiert.

Falls eine Wasserkühlung von Einzelspulen benötigt wird, kann ein Kühlsystem über einen nicht bestromten, wasserdurchflossenen Hohlleiter vorgesehen werden. Der Hohlleiter kann bei den Ringspulen 5, 6 als Wickellage außenseitig am Wickelpaket liegen und bevorzugt mit dem Wickelpaket vergossen sein. Bei den Sattelspulen 1-4 kann die Kühlleiter-Wickellage außen auf dem stromführenden Wickelpaket liegen. Bei den Flächenspulen 7-10 bzw. 7-12 wird vorteilhaft auf den Wickelkörper erst eine Hohlleiterlage gewickelt und darauf dann der stromführende Rechteckleiter.

## Patentansprüche

1. Spulenanordnung (100) zur berührungslosen Führung eines magnetischen Elementes (200), insbesondere einer Endoskopiekapsel, mit einem magnetischen Dipolmoment ($\vec{m}$) in einem Arbeitsraum (A), umfassend mehrere Einzelspulen (1-12) zur Erzeugung eines bezüglich eines kartesischen Koordinatensystems (x, y, z) beliebig orientierten Magnetfeldes ($\vec{B}$) und/oder zur Erzeugung mindestens vier unabhängiger Gradientenfelder einer Gradientenmatrix ($\underline{G}$), wobei

   - eine Vielzahl von ersten Einzelspulen (1-4) entlang der Umfangsrichtung einer Mantelfläche eines gedachten Zylinders, der sich derart entlang der z-Achse erstreckt, dass dessen Längsachse der z-Achse entspricht, hintereinander liegend angeordnet ist und
   - eine Vielzahl von zweiten Einzelspulen (5-6) an den beiden stirnseitigen Enden des Zylinders angeordnet ist,

   **dadurch gekennzeichnet, dass**

   - eine Vielzahl von dritten Einzelspulen (7-12) flügelartig angeordnet ist, wobei die jeweilige Einzelspule in der x-y-Ebene, in der x-z-Ebene oder in der y-z-Ebene angeordnet ist und wobei der geometrische Mittelpunkt einer flügelartig angeordneten Einzelspule auf einem Achsenabschnitt einer bestimmten Achse des kartesischen Koordinatensystems (x, y, z) radial außerhalb der Mantelfläche des gedachten Zylinders liegt und der Normalenvektor der Querschnittsfläche dieser flügelartig angeordneten Einzelspule senkrecht zu der bestimmten Achse steht.

2. Spulenanordnung (100) nach einem der vorhergehenden Ansprüche, **dadurch gekennzeichnet, dass** jeweils zwei Einzelspulen (1-12) einem Spulenpaar (1-2, 3-4, 5-6, 7-8, 9-10, 11-12) zugeordnet sind, wobei

- die beiden einem Spulenpaar (1-2, 3-4, 5-6, 7-8, 9-10, 11-12) zugeordneten Einzelspulen (1-12) zueinander punktsymmetrisch bezüglich des Ursprungs des kartesischen Koordinatensystems (x, y, z) angeordnet sind und

- durch die Spulenpaare (1-2, 3-4, 5-6, 7-8, 9-10, 11-12) die Komponenten ($B_x$, $B_y$, $B_z$) des Magnetfeldes ($\vec{B}$) und/oder die mindestens vier unabhängigen Gradientenfelder der symmetrischen, spurfreien Gradientenmatrix (G) erzeugbar sind.

3. Spulenanordnung (100) nach einem der vorhergehenden Ansprüche, **dadurch gekennzeichnet, dass** die ersten Einzelspulen (1-4) mindestens eine erste (1), eine zweite (2), eine dritte (3) und eine vierte (4) Spule umfassen, wobei die erste (1) und die zweite (2) Spule ein erstes Spulenpaar (1-2) und die dritte (3) und die vierte (4) Spule ein zweites Spulenpaar (3-4) bilden.

4. Spulenanordnung (100) nach Anspruch 3, **dadurch gekennzeichnet, dass**

- der geometrische Mittelpunkt der ersten Spule (1) auf dem negativen x-Achsenabschnitt liegt,
- der geometrische Mittelpunkt der zweiten Spule (2) auf dem positiven x-Achsenabschnitt liegt,
- der geometrische Mittelpunkt der dritten Spule (3) auf dem negativen y-Achsenabschnitt liegt und
- der geometrische Mittelpunkt der vierten Spule (4) auf dem positiven y-Achsenabschnitt liegt,

wobei die Querschnittsflächen der ersten (1) und der zweiten (2) Spule in Ebenen parallel zur y-z-Ebene liegen und die Querschnittsflächen der dritten (3) und der vierten (4) Spule in Ebenen parallel zur x-z-Ebene liegen.

5. Spulenanordnung (100) nach einem der Ansprüche 3 oder 4, **dadurch gekennzeichnet, dass** sich die erste (1), die zweite (2), die dritte (3) und die vierte (4) Spule entlang der Umfangsrichtung der Mantelfläche über denselben Raumwinkel ($\alpha$) erstrecken.

6. Spulenanordnung (100) nach einem der Ansprüche 3 oder 4, **dadurch gekennzeichnet, dass** sich die erste (1) und die zweite (2) Spule jeweils entlang der Umfangsrichtung der  Mantelfläche über einen ersten Raumwinkel ($\alpha_1$) und sich die dritte (3) und die vierte (4) Spule jeweils entlang der Umfangsrichtung der Mantelfläche über einen zweiten Raumwinkel ($\alpha_2$) erstrecken.

7. Spulenanordnung (100) nach Anspruch 6, **dadurch gekennzeichnet, dass** der zweite Raumwinkel ($\alpha_2$) größer ist als der erste Raumwinkel ($\alpha_1$), wobei der zweite Raumwinkel ($\alpha_2$) insbesondere in einem Bereich $90° < \alpha_2 < 110°$ liegt.

8. Spulenanordnung (100) nach einem der vorhergehenden Ansprüche, dadurch gekenntzeichnet, dass die ersten Einzelspulen (1-4) jeweils nach Bauart einer Sattelspule, nach Bauart einer Flächenspule oder nach Bauart einer Ringspule ausgebildet sind.

9. Spulenanordnung (100) nach einem der vorhergehenden Ansprüche, **dadurch gekennzeichnet, dass** die ersten Einzelspulen (1-4) in Umfangsrichtung der Mantelfläche gesehen gegenseitig beabstandet angeordnet sind.

10. Spulenanordnung (100) nach einem der vorhergehenden Ansprüche, **dadurch gekennzeichnet, dass** die zweiten Einzelspulen (5-6) mindestens eine fünfte (5) und eine sechste (6) Spule umfassen, wobei die fünfte (5) und die sechste (6) Spule ein drittes Spulenpaar (5-6) bilden.

11. Spulenanordnung (100) nach Anspruch 10, **dadurch gekennzeichnet, dass**

- der geometrische Mittelpunkt der fünften (5) Spule auf dem positiven z-Achsenabschnitt und
- der geometrische Mittelpunkt der sechsten (6) Spule auf dem negativen z-Achsenabschnitt liegt,

wobei die Querschnittsflächen der fünften (5) und der sechsten (6) Spule in Ebenen parallel zur x-y-Ebene liegen.

12. Spulenanordnung (100) nach einem der vorhergehenden Ansprüche, **dadurch gekennzeichnet, dass** die zweiten Einzelspulen (5-6) als Ringspulen oder als Flächenspulen ausgebildet sind.

13. Spulenanordnung (100) nach einem der vorhergehenden Ansprüche, **dadurch gekennzeichnet, dass** die dritten Einzelspulen (7-12) mindestens eine siebte (7), eine achte (8), eine neunte (9) und eine zehnte (10) Spule umfassen, wobei die siebte Spule (7) und die achte Spule (8) ein viertes Spulenpaar (7-8) und die neunte Spule (9) und die zehnte Spule (10) ein fünftes Spulenpaar (9-10) bilden.

**14.** Spulenanordnung (100) nach Anspruch 13, **dadurch gekennzeichnet, dass** die dritten Einzelspulen (7-12) zusätzlich mindestens eine elfte (11) und eine zwölfte (12) Spule umfassen, wobei die elfte (11) und die zwölfte (12) Spule ein sechstes Spulenpaar (11-12) bilden.

**15.** Spulenanordnung (100) nach Anspruch 14, **dadurch gekennzeichnet, dass**

- der geometrische Mittelpunkt der elften Spule (11) auf dem negativen x-Achsenabschnitt liegt,
- der geometrische Mittelpunkt der zwölften Spule (12) auf dem positiven x-Achsenabschnitt liegt,

wobei die Querschnittsflächen der elften (11) und der zwölften (12) Spule in der x-y-Ebene liegen.

**16.** Spulenanordnung (100) nach einem der Ansprüche 13 bis 15, **dadurch gekennzeichnet, dass**

- der geometrische Mittelpunkt der siebten Spule (7) auf dem negativen x-Achsenabschnitt liegt,
- der geometrische Mittelpunkt der achten Spule (8) auf dem positiven x-Achsenabschnitt liegt,
- der geometrische Mittelpunkt der neunten Spule (9) auf dem negativen y-Achsenabschnitt liegt und
- der geometrische Mittelpunkt der zehnten Spule (10) auf dem positiven y-Achsenabschnitt liegt,

wobei die Querschnittsflächen der siebten (7) und der achten (8) Spule in der x-z-Ebene und die Querschnittsflächen der neunten (9) und der zehnten (10) Spule in der x-y-Ebene liegen.

**17.** Spulenanordnung (100) nach einem der Ansprüche 14 bis 16, **dadurch gekennzeichnet, dass** die Positionen der geometrischen Mittelpunkte der elften (11) und der siebten (7) Spule sowie die Positionen der geometrischen Mittelpunkte der zwölften (12) und der achten (8) Spule deckungsgleich oder eng benachbart sind.

**18.** Spulenanordnung (100) nach einem der Ansprüche 13 bis 15, **dadurch gekennzeichnet, dass**

- der geometrische Mittelpunkt der siebten Spule (7) auf dem negativen y-Achsenabschnitt liegt,
- der geometrische Mittelpunkt der achten Spule (8) auf dem positiven y-Achsenabschnitt liegt,
- der geometrische Mittelpunkt der neunten Spule (9) auf dem negativen y-Achsenabschnitt liegt und
- der geometrische Mittelpunkt der zehnten Spule (10) auf dem positiven y-Achsenabschnitt liegt,

wobei die Querschnittsflächen der siebten (7) und der achten (8) Spule in der y-z-Ebene und die Querschnittsflächen der neunten (9) und der zehnten (10) Spule in der x-y-Ebene liegen.

**19.** Spulenanordnung (100) nach Anspruch 18, **dadurch gekennzeichnet, dass** die Positionen der geometrischen Mittelpunkte der neunten (9) und der siebten (7) Spule sowie die Positionen der geometrischen Mittelpunkte der zehnten (10) und der achten (8) Spule deckungsgleich oder eng benachbart sind.

**20.** Spulenanordnung (100) nach einem der Ansprüche 13 bis 19, **dadurch gekennzeichnet, dass** die dritten Einzelspulen (7-12) als Ringspulen oder als Flächenspulen ausgebildet sind.

**21.** Spulenanordnung (100) nach einem der nach einem der Ansprüche 13 bis 20, **dadurch gekennzeichnet, dass**

- die siebte (7) und die achte (8) Spule und/oder
- die neunte (9) und die zehnte (10) Spule und/oder
- die elfte (11) und die zwölfte (12) Spule
jeweils aus mindestens zwei in Richtung des auf der jeweiligen Querschnittsfläche der Spule stehenden Normalenvektors übereinander liegenden Teilspulen (7a, 7b, 8a, 8b,...) besteht, wobei zwischen zwei übereinander liegenden Teilspulen (7a, 7b, 8a, 8b,...) ein Zwischenraum liegt.

**22.** Spulenanordnung (100) nach einem der Ansprüche 3 bis 9, einem der Ansprüche 10 bis 12 und einem der Ansprüche 13 bis 21, **dadurch gekennzeichnet, dass**

- ein erster Leistungsverstärker zur Stromversorgung der ersten Spule (1) und der zweiten Spule (2) vorgesehen ist,
- ein zweiter Leistungsverstärker zur Stromversorgung der siebten Spule (7) und der achten Spule (8) vorgesehen ist,

- ein dritter Leistungsverstärker zur Stromversorgung der neunten Spule (9) und der zehnten Spule (10) vorgesehen ist und

- ein vierter Leistungsverstärker zur Stromversorgung der elften Spule (11) und der zwölften Spule (12) vorgesehen ist, und wobei zur Stromversorgung der dritten (3), der vierten (4), der fünften (5) und der sechsten (6) Spule jeweils ein weiterer Leistungsverstärker vorgesehen ist.

23. Spulenanordnung (100) nach einem der Ansprüche 3 bis 9, einem der Ansprüche 10 bis 12 und einem der Ansprüche 13 bis 21, **dadurch gekennzeichnet, dass**

- ein erster Leistungsverstärker zur Stromversorgung der ersten Spule (1) und der zweiten Spule (2) vorgesehen ist,

- ein zweiter Leistungsverstärker zur Stromversorgung der fünften Spule (5) und der sechsten Spule (6) vorgesehen ist,

- ein dritter Leistungsverstärker zur Stromversorgung der siebten Spule (7) und der achten Spule (8) vorgesehen ist,

- ein vierter Leistungsverstärker zur Stromversorgung der neunten Spule (9) und der zehnten Spule (10) vorgesehen ist und

und wobei zur Stromversorgung der dritten (3) und der vierten (4) Spule jeweils ein weiterer Leistungsverstärker vorgesehen ist.

24. Spulenanordnung (100) nach einem der Ansprüche 3 bis 9, einem der Ansprüche 10 bis 12 und einem der Ansprüche 13 bis 21, **dadurch gekennzeichnet, dass**

- ein erster Leistungsverstärker zur Stromversorgung der ersten Spule (1) und der zweiten Spule (2) vorgesehen ist,

- ein zweiter Leistungsverstärker zur Stromversorgung der siebten Spule (7) und der achten Spule (8) vorgesehen ist,

- ein dritter Leistungsverstärker zur Stromversorgung der neunten Spule (9) und der zehnten Spule (10) vorgesehen ist und

und wobei zur Stromversorgung der dritten (3), der vierten (4), der fünften (5) und der sechsten (6) Spule jeweils ein weiterer Leistungsverstärker vorgesehen ist.

25. Spulenanordnung (100) nach einem der Ansprüche 3 bis 9, einem der Ansprüche 10 bis 12 und einem der Ansprüche 13 bis 21, **dadurch gekennzeichnet, dass**

- ein erster Leistungsverstärker zur Stromversorgung der dritten Spule (3) und der vierten Spule (4) vorgesehen ist,

- ein zweiter Leistungsverstärker zur Stromversorgung der siebten Spule (7) und der achten Spule (8) vorgesehen ist,

- ein dritter Leistungsverstärker zur Stromversorgung der neunten Spule (9) und der zehnten Spule (10) vorgesehen ist und

und wobei zur Stromversorgung der ersten (1), der zweiten (2), der fünften (5) und der sechsten (6) Spule jeweils ein weiterer Leistungsverstärker vorgesehen ist.

26. Spulenanordnung (100) nach einem der Ansprüche 22 bis 24, **dadurch gekennzeichnet, dass** das magnetische Element (200) eine Endoskopiekapsel mit einem magnetischen Dipolmoment ($\vec{m}$) ist, welches parallel zu einer Längsachse der Endoskopiekapsel ausgerichtet ist.

27. Spulenanordnung (100) nach einem der Ansprüche 22 oder 25, **dadurch gekennzeichnet, dass** das magnetische Element (200) eine Endoskopiekapsel mit einem magnetischen Dipolmoment ($\vec{m}$) ist, welches senkrecht zu einer Längsachse der Endoskopiekapsel ausgerichtet ist.

28. Spulenanordnung (100) nach einem der vorhergehenden Ansprüche, **dadurch gekennzeichnet, dass** das magnetische Element (200) einen Permanentmagneten enthält, der fest mit dem magnetischen Element verbunden ist.

**29.** Spulenanordnung (100) nach einem der vorhergehenden Ansprüche, **dadurch gekennzeichnet, dass** der Schwerpunkt des magnetischen Elements (200) außerhalb der geometrischen Mitte des magnetischen Elements (200), insbesondere auf dessen Längsachse liegend und in Richtung der Längsachse verschoben, angeordnet ist.

**30.** Spulenanordnung (100) nach einem der vorhergehenden Ansprüche, **dadurch gekennzeichnet, dass** die Spulenanordnung (100) um die z-Achse gedreht angeordnet ist.

**Claims**

**1.** Coil arrangement (100) for contactless guidance of a magnetic element (200), in particular an endoscopy capsule, with a magnetic dipole moment ($\vec{m}$) in a work space (A), comprising multiple individual coils (1 - 12) to generate a magnetic field ($\vec{B}$) oriented arbitrarily in respect of a Cartesian coordinate system (x, y, z) and/or to generate at least four independent gradient fields of a gradient matrix ($\underline{G}$), wherein

- a plurality of first individual coils (1 - 4) is arranged lying in series along the circumferential direction of a surface shell of an imaginary cylinder extending along the z-axis such that the longitudinal axis corresponds to the z-axis and
- a plurality of second individual coils (5 - 6) is arranged at the two facing-side ends of the cylinder,

**characterised in that**

- a plurality of third individual coils (7 - 12) is arranged like blades, wherein the respective individual coil is arranged in the x-y plane, in the x-z plane or in the y-z plane and wherein the geometric centre point of an individual coil arranged in the manner of a blade lies radially outside of the surface shell of the imaginary cylinder on an axis segment of a specific axis of the Cartesian coordinate system (x, y, z) and the normal vector of the cross-sectional surface of this individual coil arranged in the manner of a blade is at right angles to the specific axis.

**2.** Coil arrangement (100) according to any of the preceding claims, **characterised in that** two respective individual coils (1 - 12) are associated with a coil pair (1 - 2, 3 - 4, 5 - 6, 7 - 8, 9 - 10, 11 - 12), wherein

- the two individual coils (1 - 12) associated with a coil pair (1 - 2, 3 - 4, 5 - 6, 7 - 8, 9 - 10, 11 - 12) are arranged point-symmetrical relative to the origin of the Cartesian coordinate system (x, y, z) and
- the components ($B_x$, $B_y$, $B_z$) of the magnetic field ($\vec{B}$) and/or the at least four independent gradient fields of the symmetrical, spurfree gradient matrix ($\underline{G}$) can be generated by the coil pairs (1 - 2, 3 - 4, 5 - 6, 7 - 8, 9 - 10, 11 - 12).

**3.** Coil arrangement (100) according to any of the preceding claims, **characterised in that** the first individual coils (1 - 4) comprise at least one first (1), one second (2), one third (3) and one fourth (4) coil, wherein the first coil (1) and the second coil (2) form a first coil pair (1 - 2) and the third coil (3) and the fourth coil (4) form a second coil pair (3 - 4).

**4.** Coil arrangement (100) according to claim 3, **characterised in that**

- the geometric centre point of the first coil (1) lies on the negative x-axis segment,
- the geometric centre point of the second coil (2) lies on the positive x-axis segment,
- the geometric centre point of the third coil (3) lies on the negative y-axis segment,
- the geometric centre point of the fourth coil (4) lies on the positive y-axis segment,

wherein the cross sectional surfaces of the first coil (1) and the second coil (2) lie in planes parallel to the y-z-plane and the cross sectional surfaces of the third coil (3) and the fourth coil (4) lie in planes parallel to the x-z-plane.

**5.** Coil arrangement (100) according to one of the claims 3 or 4, **characterised in that** the first coil (1), the second coil (2), the third coil (3) and the fourth coil (4) extend over the same solid angle ($\alpha$) along the circumferential direction of the surface shell.

**6.** Coil arrangement (100) according to any of the claims 3 or 4, **characterised in that** the first coil (1) and the second coil (2) respectively extend over a first solid angle ($\alpha_1$) along the circumferential direction of the surface shell and the third coil (3) and the fourth coil (4) respectively extend over a second solid angle ($\alpha_2$) along the circumferential

direction of the surface shell.

7. Coil arrangement (100) according to claim 6, **characterised in that** the second solid angle ($\alpha_2$) is greater than the first solid angle ($\alpha_1$), wherein the second solid angle ($\alpha_2$) in particular lies in a range of 90° < $\alpha_2$ < 110°.

8. Coil arrangement (100) according to any of the preceding claims, **characterised in that** the first individual coils (1 - 4) are fashioned in the design of a saddle coil, the design of a surface coil or the design of a ring coil.

9. Coil arrangement (100) according to any of the preceding claims, **characterised in that** the first individual coils (1 - 4) are arranged mutually spaced apart as viewed in the circumferential direction of the surface shell.

10. Coil arrangement (100) according to any of the preceding claims, **characterised in that** the second individual coils (5 - 6) form at least one fifth coil (5) and one sixth coil (6), wherein the fifth coil (5) and the sixth coil (6) form a third coil pair (5-6) .

11. Coil arrangement (100) according to claim 10, **characterised in that**

- the geometric centre point of the fifth coil (5) lies on the positive z-axis segment and
- the geometric centre point of the sixth coil (6) lies on the negative z-axis segment,

wherein the cross sectional surfaces of the fifth coil (5) and the sixth coil (6) lie in planes parallel to the x-y-plane.

12. Coil arrangement (100) according to any of the preceding claims, **characterised in that** the second individual coils (5 - 6) are fashioned as ring coils or as surface coils.

13. Coil arrangement (100) according to any of the preceding claims, **characterised in that** the third individual coils (7 - 12) comprise at least one seventh coil (7), one eighth coil (8), one ninth coil (9) and one tenth coil (10), wherein the seventh coil (7) and the eighth coil (8) form a fourth coil pair (7 - 8) and the ninth coil (9) and the tenth coil (10) form a fifth coil pair (9-10) .

14. Coil arrangement (100) according to claim 13, **characterised in that** the third individual coils (7 - 12) additionally comprise at least one eleventh coil (11) and one twelfth coil (12), wherein the eleventh coil (11) and the twelfth coil (12) form a sixth coil pair (11 - 12).

15. Coil arrangement (100) according to claim 14, **characterised in that**

- the geometric centre point of the eleventh coil (11) lies on the negative x-axis segment,
- the geometric centre point of the twelfth coil (12) lies on the positive x-axis segment,

wherein the cross sectional surfaces of the eleventh coil (11) and the twelfth coil (12) lie in the x-y-plane.

16. Coil arrangement (100) according to any of the claims 13 through 15, **characterised in that**

- the geometric centre point of the seventh coil (7) lies on the negative x-axis segment,
- the geometric centre point of the eighth coil (8) lies on the positive x-axis segment,
- the geometric centre point of the ninth coil (9) lies on the negative y-axis segment,
- the geometric centre point of the tenth coil (10) lies on the positive y-axis segment,

wherein the cross sectional surfaces of the seventh coil (7) and the eighth coil (8) lie in the x-z-plane and the cross sectional surfaces of the ninth coil (9) and the tenth coil (10) lie in the x-y-plane.

17. Coil arrangement (100) according to any of the claims 14 through 16, **characterised in that** the positions of the geometric centre points of the eleventh coil (11) and the seventh coil (7) as well as the positions of the geometric centre points of the twelfth coil (12) and the eighth coil (8) are congruent or closely adjacent.

18. Coil arrangement (100) according to any of the claims 13 through 15, **characterised in that**

- the geometric centre point of the seventh coil (7) lies on the negative y-axis segment,

- the geometric centre point of the eighth coil (8) lies on the positive y-axis segment,
- the geometric centre point of the ninth coil (9) lies on the negative y-axis segment,
- the geometric centre point of the tenth coil (10) lies on the positive y-axis segment,

wherein the cross sectional surfaces of the seventh coil (7) and the eighth coil (8) lie in the y-z-plane and the cross sectional surfaces of the ninth coil (9) and the tenth coil (10) lie in the x-y-plane.

19. Coil arrangement (100) according to claim 18, **characterised in that** the positions of the geometric centre points of the ninth coil (9) and the seventh coil (7) as well as the positions of the geometric centre points of the tenth coil (10) and the eighth coil (8) are congruent or closely adjacent.

20. Coil arrangement (100) according to any of the claims 13 through 19, **characterised in that** the third individual coils (7 - 12) are fashioned as ring coils or as surface coils.

21. Coil arrangement (100) according to any of according to any of [sic] the claims 13 through 20, **characterised in that**

- the seventh coil (7) and the eighth coil (8) and/or
- the ninth coil (9) and the tenth coil (10) and/or
- the eleventh coil (11) and the twelfth coil (12) respectively consists of at least two sub-coils (7a, 7b, 8a, 8b, ...) lying atop one another in the direction of the normal vector standing at the respective cross sectional surface of the coil, wherein an interstice lies between two sub-coils (7a, 7b, 8a, 8b, ...) situated one atop the other.

22. Coil arrangement (100) according to any of the claims 3 through 9, any of the claims 10 through 12 and any of the claims 13 through 21, **characterised in that**

- a first power amplifier is provided to supply power to the first coil (1) and the second coil (2),
- a second power amplifier is provided to supply power to the seventh coil (7) and the eighth coil (8),
- a third power amplifier is provided to supply power to the ninth coil (9) and the tenth coil (10),
- a fourth power amplifier is provided to supply power to the eleventh coil (11) and the twelfth coil (12),

and wherein a respective additional power amplifier is provided to supply power to the third coil (3), the fourth coil (4), the fifth coil (5) and the sixth coil (6).

23. Coil arrangement (100) according to any of the claims 3 through 9, any of the claims 10 through 12 and any of the claims 13 through 21, **characterised in that**

- a first power amplifier is provided to supply power to the first coil (1) and the second coil (2),
- a second power amplifier is provided to supply power to the fifth coil (5) and the sixth coil (6),
- a third power amplifier is provided to supply power to the seventh coil (7) and the eighth coil (8),
- a fourth power amplifier is provided to supply power to the ninth coil (9) and the tenth coil (10), and [sic]

and wherein a respective additional power amplifier is provided to supply power to the third coil (3) and the fourth coil (4).

24. Coil arrangement (100) according to any of the claims 3 through 9, any of the claims 10 through 12 and any of the claims 13 through 21, **characterised in that**

- a first power amplifier is provided to supply power to the first coil (1) and the second coil (2),
- a second power amplifier is provided to supply power to the seventh coil (7) and the eighth coil (8),
- a third power amplifier is provided to supply power to the ninth coil (9) and the tenth coil (10), and [sic]

and wherein a respective additional power amplifier is provided to supply power to the third coil (3), the fourth coil (4), the fifth coil (5) and the sixth coil (6).

25. Coil arrangement (100) according to any of the claims 3 through 9, any of the claims 10 through 12 and any of the claims 13 through 21, **characterised in that**

- a first power amplifier is provided to supply power to the third coil (3) and the fourth coil (4),

- a second power amplifier is provided to supply power to the seventh coil (7) and the eighth coil (8),
- a third power amplifier is provided to supply power to the ninth coil (9) and the tenth coil (10), and [sic]

and wherein a respective additional power amplifier is provided to supply power to the first coil (1), the second coil (2), the fifth coil (5) and the sixth coil (6).

26. Coil arrangement (100) according to any of the claims 22 through 24, **characterised in that** the magnetic element (200) is an endoscopy capsule with a magnetic dipole moment ($\overline{m}$) which is aligned parallel to a longitudinal axis of the endoscopy capsule.

27. Coil arrangement (100) according to any of the claims 22 through 25, **characterised in that** the magnetic element (200) is an endoscopy capsule with a magnetic dipole moment ($\overline{m}$) which is aligned perpendicular to a longitudinal axis of the endoscopy capsule.

28. Coil arrangement (100) according to any of the preceding claims, **characterised in that** the magnetic element (200) contains a permanent magnet that is firmly connected with the magnetic element.

29. Coil arrangement (100) according to any of the preceding claims, **characterised in that** the focal point of the magnetic element (200) is arranged outside of the geometric centre of the magnetic element (200), in particular situated on its longitudinal axis and shifted in the direction of the longitudinal axis.

30. Coil arrangement (100) according to any of the preceding claims, **characterised in that** the coil arrangement (100) is arranged rotated around the z-axis.

**Revendications**

1. Dispositif de bobines (100) pour le guidage sans contact d'un élément magnétique (200), notamment d'une capsule endoscopique, présentant un moment dipolaire magnétique (m) dans un espace de travail (A), comprenant plusieurs bobines individuelles (1-12) destinées à générer un champ magnétique ($\vec{B}$) orienté de manière quelconque par rapport à un système de coordonnées cartésiennes (x, y, z) et/ou à générer au moins quatre champs de gradients indépendants d'une matrice de gradients ($\underline{G}$),

   - une pluralité de premières bobines individuelles (1-4) étant situées les unes derrière les autres le long de la direction circonférentielle d'une surface latérale d'un cylindre imaginaire qui s'étend le long de l'axe z de telle sorte que son axe longitudinal corresponde à l'axe z et
   - une pluralité de deuxièmes bobines individuelles (5-6) étant situées au niveau des deux extrémités frontales du cylindre,

   **caractérisé en ce que**

   - une pluralité de troisièmes bobines individuelles (7-12) étant disposées comme des ailes, la bobine individuelle respective étant située dans le plan x-y, dans le plan x-z ou dans le plan y-z et le centre géométrique d'une bobine individuelle disposée comme une aile étant situé sur une portion d'axe d'un axe déterminé du système de coordonnées cartésiennes (x, y, z) à l'extérieur, dans le sens radial, de la surface latérale du cylindre imaginaire et le vecteur normal de l'aire de section de cette bobine individuelle disposée comme une aile étant perpendiculaire à l'axe déterminé.

2. Dispositif de bobines (100) selon l'une des revendications précédentes, **caractérisé en ce que** deux bobines individuelles (1-12) sont à chaque fois associées à une paire de bobines (1-2, 3-4, 5-6, 7-8, 9-10, 11-12),

   - les deux bobines individuelles (1-12) associées à une paire de bobines (1-2, 3-4, 5-6, 7-8, 9-10, 11-12) présentant les unes par rapport aux autres une symétrie ponctuelle par rapport à l'origine du système de coordonnées cartésiennes (x, y, z) et
   - les composantes ($B_x$, $B_y$, $B_z$) du champ magnétique ($\overline{B}$) et/ou les au moins quatre champs de gradients indépendants de la matrice de gradients symétrique sans trace ($\underline{G}$) pouvant être générés par les paires de bobines (1-2, 3-4, 5-6, 7-8, 9-10, 11-12).

**3.** Dispositif de bobines (100) selon l'une des revendications précédentes, **caractérisé en ce que** les premières bobines individuelles (1-4) comprennent au moins une première (1), une deuxième (2), une troisième (3) et une quatrième (4) bobine, la première (1) et la deuxième (2) bobine formant une première paire de bobines (1-2) et la troisième (3) et la quatrième (4) bobine formant une deuxième paire de bobines (3-4).

**4.** Dispositif de bobines (100) selon la revendication 3, **caractérisé en ce que**

- le centre géométrique de la première bobine (1) est situé sur la portion négative de l'axe x,
- le centre géométrique de la deuxième bobine (2) est situé sur la portion positive de l'axe x,
- le centre géométrique de la troisième bobine (3) est situé sur la portion négative de l'axe y et
- le centre géométrique de la quatrième bobine (4) est situé sur la portion positive de l'axe y,

les aires de section de la première (1) et de la deuxième (2) bobine étant situées dans des plans parallèles au plan y-z et les aires de section de la troisième (3) et de la quatrième (4) bobine étant situées dans des plans parallèles au plan x-z.

**5.** Dispositif de bobines (100) selon l'une des revendications 3 ou 4, **caractérisé en ce que** la première (1), la deuxième (2), la troisième (3) et la quatrième (4) bobine s'étendent sur le même angle solide ($\alpha$) le long de la direction circonférentielle de la surface latérale.

**6.** Dispositif de bobines (100) selon l'une des revendications 3 ou 4, **caractérisé en ce que** la première (1) et la deuxième (2) bobine s'étendent respectivement sur un premier angle solide ($\alpha_1$) le long de la direction circonférentielle de la surface latérale et la troisième (3) et la quatrième (4) bobine s'étendent à chaque fois sur un second angle solide ($\alpha_2$) le long de la direction circonférentielle de la surface latérale.

**7.** Dispositif de bobines (100) selon la revendication 6, **caractérisé en ce que** le second angle solide ($\alpha_2$) est supérieur au premier angle solide ($\alpha_1$), le second angle solide ($\alpha_2$) étant notamment compris dans une plage $90° < \alpha_2 < 110°$.

**8.** Dispositif de bobines (100) selon l'une des revendications précédentes, **caractérisé en ce que** les premières bobines individuelles (1-4) sont exécutées à chaque fois sur le modèle d'une bobine en sellette, sur le modèle d'une bobine plate ou sur le modèle d'une bobine annulaire.

**9.** Dispositif de bobines (100) selon l'une des revendications précédentes, **caractérisé en ce que** les premières bobines individuelles (1-4) sont mutuellement espacées, vues dans la direction circonférentielle de la surface latérale.

**10.** Dispositif de bobines (100) selon l'une des revendications précédentes, **caractérisé en ce que** les deuxièmes bobines individuelles (5-6) comprennent au moins une cinquième (5) et une sixième (6) bobine, la cinquième (5) et la sixième (6) bobine formant une troisième paire de bobines (5-6).

**11.** Dispositif de bobines (100) selon la revendication 10, **caractérisé en ce que**

- le centre géométrique de la cinquième (5) bobine est situé sur la portion positive de l'axe z et
- le centre géométrique de la sixième (6) bobine est situé sur la portion négative de l'axe z,

les aires de section de la cinquième (5) et de la sixième (6) bobine étant situées dans des plans parallèles au plan x-y.

**12.** Dispositif de bobines (100) selon l'une des revendications précédentes, **caractérisé en ce que** les deuxièmes bobines individuelles (5-6) sont exécutées en tant que bobines annulaires ou en tant que bobines plates.

**13.** Dispositif de bobines (100) selon l'une des revendications précédentes, **caractérisé en ce que** les troisièmes bobines individuelles (7-12) comprennent au moins une septième (7), une huitième (8), une neuvième (9) et une dixième (10) bobine, la septième bobine (7) et la huitième bobine (8) formant une quatrième paire de bobines (7-8) et la neuvième bobine (9) et la dixième bobine (10) formant une cinquième paire de bobines (9-10).

**14.** Dispositif de bobines (100) selon la revendication 13, **caractérisé en ce que** les troisièmes bobines individuelles (7-12) comprennent, en plus, au moins une onzième (11) et une douzième (12) bobine, la onzième (11) et la douzième (12) bobine formant une sixième paire de bobines (11-12).

**15.** Dispositif de bobines (100) selon la revendication 14, **caractérisé en ce que**

- le centre géométrique de la onzième bobine (11) est situé sur la portion négative de l'axe x,
- le centre géométrique de la douzième bobine (12) est situé sur la portion positive de l'axe x,

les aires de section de la onzième (11) et de la douzième (12) bobine étant situées dans le plan x-y.

**16.** Dispositif de bobines (100) selon l'une des revendications 13 à 15, **caractérisé en ce que**

- le centre géométrique de la septième bobine (7) est situé sur la portion négative de l'axe x,
- le centre géométrique de la huitième bobine (8) est situé sur la portion positive de l'axe x,
- le centre géométrique de la neuvième bobine (9) est situé sur la portion négative de l'axe y et
- le centre géométrique de la dixième bobine (10) est situé sur la portion positive de l'axe y,

les aires de section de la septième (7) et de la huitième (8) bobine étant situées dans le plan x-z et les aires de section de la neuvième (9) et de la dixième (10) bobine étant situées dans le plan x-y.

**17.** Dispositif de bobines (100) selon l'une des revendications 14 à 16, **caractérisé en ce que** les positions des centres géométriques de la onzième (11) et de la septième (7) bobine ainsi que les positions des centres géométriques de la douzième (12) et de la huitième (8) bobine coïncident ou sont très proches l'une de l'autre.

**18.** Dispositif de bobines (100) selon l'une des revendications 13 à 15, **caractérisé en ce que**

- le centre géométrique de la septième bobine (7) est situé sur la portion négative de l'axe y,
- le centre géométrique de la huitième bobine (8) est situé sur la portion positive de l'axe y,
- le centre géométrique de la neuvième bobine (9) est situé sur la portion négative de l'axe y et
- le centre géométrique de la dixième bobine (10) est situé sur la portion positive de l'axe y,

les aires de section de la septième (7) et de la huitième (8) bobine étant situées dans le plan y-z et les aires de section de la neuvième (9) et de la dixième (10) bobine étant situées dans le plan x-y.

**19.** Dispositif de bobines (100) selon la revendication 18, **caractérisé en ce que** les positions des centres géométriques de la neuvième (9) et de la septième (7) bobine ainsi que les positions des centres géométriques de la dixième (10) et de la huitième (8) bobine coïncident ou sont très proches l'une de l'autre.

**20.** Dispositif de bobines (100) selon l'une des revendications 13 à 19, **caractérisé en ce que** les troisièmes bobines individuelles (7-12) sont exécutées en tant que bobines annulaires ou en tant que bobines plates.

**21.** Dispositif de bobines (100) selon l'une des revendications 13 à 20, **caractérisé en ce que**

- la septième (7) et la huitième (8) bobine et/ou
- la neuvième (9) et la dixième ((10) bobine et/ou
- la onzième (11) et la douzième (12) bobine est/sont formée/s à chaque fois d'au moins deux sections de bobine (7a, 7b, 8a, 8b,...) disposées l'une au-dessus de l'autre dans la direction du vecteur normal à l'aire de section respective de la bobine, un espace intermédiaire étant situé entre deux sections de bobine (7a, 7b, 8a, 8b,...) disposées l'une au-dessus de l'autre.

**22.** Dispositif de bobines (100) selon l'une des revendications 3 à 9, l'une des revendications 10 à 12 et l'une des revendications 13 à 21, **caractérisé en ce que**

- un premier amplificateur de puissance est prévu pour l'alimentation de la première bobine (1) et de la deuxième bobine (2),
- un deuxième amplificateur de puissance est prévu pour l'alimentation de la septième bobine (7) et de la huitième bobine (8),
- un troisième amplificateur de puissance est prévu pour l'alimentation de la neuvième bobine (9) et de la dixième bobine (10) et
- un quatrième amplificateur de puissance est prévu pour l'alimentation de la onzième bobine (11) et de la douzième bobine (12),

et un amplificateur de puissance supplémentaire étant à chaque fois prévu pour l'alimentation de la troisième (3), de la quatrième (4), de la cinquième (5) et de la sixième (6) bobine.

**23.** Dispositif de bobines (100) selon l'une des revendications 3 à 9, l'une des revendications 10 à 12 et l'une des revendications 13 à 21, **caractérisé en ce que**

- un premier amplificateur de puissance est prévu pour l'alimentation de la première bobine (1) et de la deuxième bobine (2),
- un deuxième amplificateur de puissance est prévu pour l'alimentation de la cinquième bobine (5) et de la sixième bobine (6),
- un troisième amplificateur de puissance est prévu pour l'alimentation de la septième bobine (7) et de la huitième bobine (8),
- un quatrième amplificateur de puissance est prévu pour l'alimentation de la neuvième bobine (9) et de la dixième bobine (10) et

un amplificateur de puissance supplémentaire étant à chaque fois prévu pour l'alimentation de la troisième (3) et de la quatrième (4) bobine.

**24.** Dispositif de bobines (100) selon l'une des revendications 3 à 9, l'une des revendications 10 à 12 et l'une des revendications 13 à 21, **caractérisé en ce que**

- un premier amplificateur de puissance est prévu pour l'alimentation de la première bobine (1) et de la deuxième bobine (2),
- un deuxième amplificateur de puissance est prévu pour l'alimentation de la septième bobine (7) et de la huitième bobine (8),
- un troisième amplificateur de puissance est prévu pour l'alimentation de la neuvième bobine (9) et de la dixième bobine (10),

et un amplificateur de puissance supplémentaire étant à chaque fois prévu pour l'alimentation de la troisième (3), de la quatrième (4), de la cinquième (5) et de la sixième (6) bobine.

**25.** Dispositif de bobines (100) selon l'une des revendications 3 à 9, l'une des revendications 10 à 12 et l'une des revendications 13 à 21, **caractérisé en ce que**

- un premier amplificateur de puissance est prévu pour l'alimentation de la troisième bobine (3) et de la quatrième bobine (4),
- un deuxième amplificateur de puissance est prévu pour l'alimentation de la septième bobine (7) et de la huitième bobine (8),
- un troisième amplificateur de puissance est prévu pour l'alimentation de la neuvième bobine (9) et de la dixième bobine (10),

et un amplificateur de puissance supplémentaire étant à chaque fois prévu pour l'alimentation de la première (1), de la deuxième (2), de la cinquième (5) et de la sixième (6) bobine.

**26.** Dispositif de bobines (100) selon l'une des revendications 22 à 24, **caractérisé en ce que** l'élément magnétique (200) est une capsule endoscopique présentant un moment dipolaire magnétique $(\overline{m})$ qui est orienté parallèlement à un axe longitudinal de la capsule endoscopique.

**27.** Dispositif de bobines (100) selon l'une des revendications 22 ou 25, **caractérisé en ce que** l'élément magnétique (200) est une capsule endoscopique présentant un moment dipolaire magnétique $(\overline{m})$ qui est orienté perpendiculairement à un axe longitudinal de la capsule endoscopique.

**28.** Dispositif de bobines (100) selon l'une des revendications précédentes, **caractérisé en ce que** l'élément magnétique (200) contient un aimant permanent qui est relié de manière fixe à l'élément magnétique.

**29.** Dispositif de bobines (100) selon l'une des revendications précédentes, **caractérisé en ce que** le centre de gravité de l'élément magnétique (200) se trouve à l'extérieur du centre géométrique de l'élément magnétique (200), il est notamment situé sur son axe longitudinal et déplacé dans la direction de l'axe longitudinal.

**30.** Dispositif de bobines (100) selon l'une des revendications précédentes, **caractérisé en ce que** le dispositif de bobines (100) tourne autour de l'axe z.

FIG 1

FIG 2A

FIG 2B

FIG 2C

FIG 3A

FIG 3B

FIG 3C

FIG 4A

FIG 4B

## FIG 5A

## FIG 5B

FIG 6

FIG 7

FIG 8A

FIG 8B

FIG 8C

FIG 8D

FIG 8E

FIG 8F

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10340925 B3
- WO 2006092421 A1